# EUROPÄISCHE PATENTANMELDUNG

(11) **EP 0 747 060 A2**
(43) Veröffentlichungstag der Anmeldung: **11.12.1996**
(21) Anmeldenummer: 96890096.9
(22) Anmeldetag: 04.06.1996
(51) Int. Cl.: A61K 39/395, C07K 16/36

(54) **Anti-plasma-Antikörper-Präparation**

(30) Priorität: 09.06.1995 AT 987/95
(71) Anmelder: IMMUNO Aktiengesellschaft, A-1221 Wien (AT)
(72) Erfinder: Eibl, Johann, Dr., A-1180 Wien (AT); Turecek, Peter, Dr., A-3400 Klosterneuburg Weidling (AT); Schwarz, Hans Peter, Doz. Dr., A-1180 Wien (AT)
(74) Vertreter: Alge, Daniel

(57) **Zusammenfassung**

Beschrieben wird ein anti-Plasma-Antikörperpräparat zur Behandlung an einem Säugetier, welches Präparat mehrere Blutfaktoren direkt oder indirekt inhibieren und/oder eliminieren kann, ein Verfahren zur Herstellung eines solchen Präparats sowie ein Verfahren zur Evaluierung von Substanzen zur Behandlung von Gerinnungsstörungen unter Verwendung des anti-Plasma-Antikörperkörperpräparates.

Weiters wird ein Verfahren zur Bestimmung der Blutungscharakteristik eines Säugetiers beschrieben.

## Beschreibung

Die vorliegende Erfindung betrifft eine Antiplasma- und Antikörperpräparation, Verfahren zu deren Herstellung sowie die Verwendung dieser Präparationen. Weiters betrifft die Erfindung ein Verfahren zur Evaluierung von Substanzen zur Behandlung von Blutgerinnungsstörungen an einem Tiermodell zusammen mit einem Verfahren zur Bestimmung der Blutungscharakteristik in einem Säugetier.

Zur Evaluierung von Substanzen bezüglich ihrer Tauglichkeit zur Behandlung von Blutgerinnungsstörungen ist es notwendig, ein geeignetes Tiermodell zur Verfügung zu stellen, welches den Charakteristika der entsprechenden klinischen Syndrome beim Menschen möglichst nahekommt.

Bisher wurden für die präklininischen Untersuchungen zu potentiellen Pharmazeutika zur Behandlung von Hämophilie beispielsweise hämophile Hunde verwendet, welche einen genetischen Defekt ähnlich dem von hämophilen Menschen aufweisen. Die Züchtungsbedingungen für diese hämophilen Hunde sind jedoch äußerst kompliziert und arbeitsintensiv, da die Hunde auf Grund ihres genetischen Defekts extrem blutungsanfällig sind. Außerdem ist die Zahl verfügbarer Tiere durch die Wahrscheinlichkeit des Auftretens des heriditären Gerinnungsdefektes begrenzt.

In J.Clin.Invest. 82 (1988), 206-211, wird beschrieben, daß ein Tiermodell für Hämophilie A durch Infusion von menschlichem anti-Faktor VIII-Antikörper in normale Kaninchen erhalten werden kann. In diesem Modell konnte durch einen hochtitrigen menschlichen anti-Faktor VIII-Antikörper innerhalb von 30 Minuten die Faktor VIII-Konzentration im Blut der Kaninchen vom normalen Wert auf unter die Nachweisgrenze gesenkt werden. Die solcherart präparierten Tiere zeigten einen starken Blutverlust, wenn eine Blutung an der Ohrarterie induziert wurde. Eine solche starke arterielle Blutung ist jedoch nicht typisch für die Blutungssituation bei Hämophilen, da dort die Blutung eher einer arteriovenösen Sickerblutung entspricht.

In der WO 95/01570 wurde dieses Tiermodell dahingehend weiterentwickelt, daß zunächst Antikörper gegen die leichte Kette des Faktor VIII:C-Moleküls aus Menschen oder Schweinen in einem ersten Tier erzeugt worden sind und anschließend mit dem erhaltenen, gereinigten monospezifischen Antikörper eine temporäre Hämophilie in einem zweiten Tier induziert wurde. Es wurde also die Spezifität der Antikörper erheblich eingeschränkt (nur auf die leichte Kette des Faktor VIII:C-Moleküls). Als Resultat erhielt man Tiere, mit sehr hohen Inhibitortitern (zwischen 17 und 42 Bethesda-Einheiten), jedoch hielt der induzierte hämophile Zustand bei den Tieren nur sehr kurz an, weshalb die Bestimmung der Nagelhautblutungszeit zur Evaluierung der Testsubstanzen schon nach 40 Minuten, und nicht, wie vorzugsweise, nach 60 Minuten, begonnen werden mußte.

Das in der WO 95/01570 beschriebene Tiermodell auf Grundlage von monospezifischen Antikörpern ist ebenfalls nur bedingt mit den tatsächlichen Begebenheiten in hämophilen Patienten vergleichbar, da in solchen Patienten in der Regel eine große Bandbreite von Inhibitorantikörpern auftreten und nicht nur Antikörper, welche gegen eine bestimmte Proteinkette oder ein bestimmtes Epitop gerichtet sind. Die kurze Zeitdauer, während der sich die Tiere gemäß der WO 95/01570 in einem für die Evaluierung der Testsubstanzen geeigneten Zustand befinden, ist für umfassende Testserien oft nicht ausreichend, weshalb es zu verfälschten Ergebnissen kommen kann, insbesondere, was den Endpunkt der Blutung anbelangt, welcher mit dem Abklingen der induzierten Hämophilie im Versuchstier interferieren kann.

Es ist daher die Aufgabe der vorliegenden Erfindung, ein verbessertes Testsystem für Substanzen zur Behandlung von Gerinnungsstörungen zur Verfügung zu stellen, welches die klinische Situation im Patienten so praxisnahe wie möglich widerspiegelt und daher bezüglich der Aussagekraft der erhaltenen Evaluierungsergebnisse eine hohe Zuverlässigkeit aufweist.

Die Aufgabe wird erfindungsgemäß gelöst durch eine anti-Plasma-Antikörperpräparation zur Behandlung an einem Säugetier, welche Präparation mehrere Blutfaktoren direkt oder indirekt inhibieren und/oder eliminieren kann.

Die Behandlung eines Säugetieres mit der erfindungsgemäßen Präparation induziert im behandelten Säugetier eine temporäre Gerinnungsstörung, die aufgrund der Spezifität bzw. Wirkung bezüglich mehrerer Blutfaktoren den Zustand im Patienten mit Blutgerinnungsstörungen, wie Hämophilie, Hemmkörperhämophilie, von Willebrand-Disease (von Willebrand-Jürgens-Syndrom) oder in Patienten mit einem Mangel an Vitamin K abhängigen Blutfaktoren praxisnahe widerspiegeln. Insbesondere ist das erfindungsgemäße System geeignet, Patienten zu simulieren, die Antikörper entwickelt haben, welche die Wirkung von Blutfaktoren, wie z.B. von Willebrand-Faktor oder Faktor VIII, inhibieren.

Ein Charakteristikum der erfindungsgemäßen Präparation ist, daß nicht - wie im Stand der Technik - nur ein monospezifischer Antikörper, welcher nur gegen einen Blutgerinnungsfaktor oder gar nur ein spezifisches Epitop dieses Blutgerinnungsfaktors gerichtet ist, zur Induktion der Blutgerinnungsstörung eingesetzt wird, sondern daß die Wirkung der Präparation mehrere Blutgerinnungsfaktoren betrifft.

Diese Wirkung kann dadurch erzielt werden, daß die Präparation entweder Antikörper gegen einen vorzugsweise humanen Blutfaktor enthält, welcher selbst unmittelbar andere Blutfaktoren inhibiert bzw. eliminiert, oder dadurch, daß die Präparation durch Immunisierung gegen eine Vielzahl von verschiedensten Epitopen, zumeist unterschiedlicher Blutfaktoren, erhalten worden ist.

Ein bevorzugtes Beispiel für den ersten Fall ist eine Präparation, enthaltend Antikörper gegen den menschlichen von Willebrand-Faktor, welcher Antikörper im Testmodell direkt mit dem tierischen von Willebrand-Faktor kreuzreagiert und indirekt den tierischen Faktor VIII destabilisiert und dadurch eliminiert. Bevorzugte Beispiele für den zweiten Fall sind eine Präparation, enthaltend Antikörper gegen den Faktor VIII/von Willebrand-Faktor-Komplex oder gegen Vitamin K abhängige Blutfaktoren, z.B. Faktoren des Prothrombin-Komplexes.

Weitere bevorzugte Beispiele betreffen Präparationen mit Antikörpern gegen Prokoagulantien (Blutgerinnungsfaktoren II, V, VII, IX, X, XI, XII, Prekallikrein, Kininogen und Gewebefaktor) aber auch Präparationen mit Antikörpern gegen Antikoagulantien (Protein C, Protein S, Antithrombin III, Heparin-Cofaktor II), Gerinnselstruktur-Faktoren (Fibrinogen und Faktor XIII), Fibrinolyse£aktoren (Plasminogen, t-PA, PAI-1 und α₂-Plasmininhibitor) und Phospholipide.

Die Eignung der erfindungsgemäßen Antikörper-Präparation für die Präparation eines Testtieres als Tiermodell kann durch verschiedene Tests beurteilt werden. Es wird vorzugsweise ein in vitro-Test durchgeführt, bei dem die Antikörper-Präparation mit einer Blut- oder Plasmaprobe eines Testtieres inkubiert wird und die Inhibierung bzw. Eliminierung der Blutfaktoren bestimmt wird. Der gewünschte Effekt einer veränderten Blutungscharakteristik kann auch in vivo durch eine Kontrollblutung am Testtier gezeigt lasmininhibitor) und Phospholipide.

Die Eignung der erfindungsgemäßen Antikörper-Präparation für die Präparation eines Testtieres als Tiermodell kann durch verschiedene Tests beurteilt werden. Es wird vorzugsweise ein in vitro-Test durchgeführt, bei dem die Antikörper-Präparation mit einer Blut- oder Plasmaprobe eines Testtieres inkubiert wird und die Inhibierung bzw. Eliminierung der Blutfaktoren bestimmt wird. Der gewünschte Effekt einer veränderten Blutungscharakteristik kann auch in vivo durch eine Kontrollblutung am Testtier gezeigt werden. Der induzierte Effekt soll vorzugsweise über längere Zeit andauern, zumindest über mehrere Stunden. Als Selektionskriterium für die erfindungsgemäße Präparation dient aber nicht nur ihre Wirksamkeit in vitro oder in vivo, sondern auch ein hoher Antikörpertiter, z.B. mehr als 300 BU/ml für Antikörper gegen Faktor VIII.

Wichtig ist stets, daß die Präparation polyklonale, polyspezische Antikörper mit möglichst breitem Epitopspektrum aufweist, welche Antikörper mit den entsprechenden Antigenen im Testtier kreuzreagieren. Die Verabreichung eines erfindungsgemäßen Präparats an ein Versuchstier ermöglicht dabei nicht nur eine qualitative Ähnlichkeit mit den menschlichen Blutgerinnungsstörungen sondern überraschenderweise auch eine längere Zeitdauer des Zustandes der induzierten Blutgerinnungsstörung beim Tier. Dies hat zur Folge, daß die Verabreichung von Testsubstanzen zur Behebung der Blutgerinnungsstörung über einen längeren Zeitraum zuverlässig evaluiert werden kann, ohne daß man damit rechnen muß, daß die Wiederherstellung des Normalzustandes beim Tier die Evaluierungsergebnisse verfälscht.

Während beim Tiermodell mit monospezifisch induziertem Faktor VIII-Mangel gemäß der WO 95/01570 trotz hohem Inhibitoriter schon nach 30 Minuten die Blutung bei 40 % der Tiere (zwei von fünf) zum Erliegen kommt, können mit der erfindungsgemäßen Präparation problemlos Blutungszeiten von 30 Minuten (90 Minuten ab Induktion mit der Antikörperpräparation) oder mehr bis zur maximal möglichen Blutungszeit erzielt werden.

Die erfindungsgemäße Präparation ist beispielsweise erhältlich durch Immunisieren eines Säugetieres mit Plasma, vorzugsweise mit menschlichem Plasma, oder einer Plasmafraktion oder mit deren rekombinantem Äquivalent, Gewinnen des Antiplasmas oder Antiserums und anschließender Absorption von einem oder mehreren Antikörpern des Antiplasmas oder des Antiserums, so daß die Präparation nur solche funktionellen Antikörper enthält, die selektiv einen oder mehrere ausgewählte Blutfaktoren in einem Säugetier funktionell inhibieren und/oder eliminieren können.

In einer bevorzugten Ausführungsform der vorliegenden Erfindung wird die Immunisierung mit Faktor VIII/von Willebrand-Komplex erzielt und die Absorption von Faktor VIII-Antikörpern durch Zugabe von Faktor VIII, insbesondere von einem Faktor VIII-Konzentrat, gewährleistet. Mit einer solcherart hergestellten Präparation lassen sich in Versuchstieren die klinische Situation des von Willebrand-Jürgens-Syndroms oder andere der Hämophilie verwandte Blutungsneigungen exakt simulieren, so daß in einem derartigen Modell Substanzen mit klinisch relevanter Aussagekraft auf ihre Wirksamkeit zur Therapie von dabei auftretenden hämorrhagischen Diäthesen untersucht werden können. Gleichfalls kann auch durch Zugabe eines von Willebrand-Faktor-Präparates zu der Präparation die spezifische Absorption der von Willebrand-Faktor-Antikörper unter geeigneten Bedingungen erfolgen, wodurch dann Antikörper gegen den Faktor VIII wirksam werden.

Ein Verfahren zur Herstellung der erfindungsgemäßen Präparation ist durch die Schritte:
- Immunisieren eines Säugetieres mit Plasma oder einer Plasmafraktion oder mit deren rekombinantem Äquivalent
- Gewinnen des Antiplasmas oder Antiserums
- gegebenenfalls Aufbereiten der Antikörperfraktion aus dem Antiplasma oder dem Antiserum
- gegebenenfalls Absorbieren von einem oder mehreren Antikörpern
   durch Inkontaktbringen der Antikörper mit bestimmten Proteinen
   und
- Fertigstellen zu einer zur Infusion in einem Säugetier geeigneten Präparation.

Bevorzugte Plasmafraktionen zur Immunisierung sind Präparationen enthaltend Faktor VIII/von Willebrand-Komplex, von Willebrand-Faktor, Prothrombinkomplex, Blutgerinnungsfaktorpräparate, etc. Die zur Immunisierung verwendeten Substanzen sind vorzugsweise humanen Ursprungs, da die zu evaluierenden Substanzen zur Behandlung am Menschen bestimmt sind. Bei Verwendung von tierischem Immunisierungsmaterial muß darauf geachtet werden, daß eine ausreichende Homologie zu den menschlichen Epitopen vorliegt.

Das Gewinnen des Antiplasmas oder Antiserums kann auf bekannte Art erfolgen, also durch Plasmapherese, Plasmaseparation (Membranfiltration, Zentrifugation, Zellseparation), Fällung, etc.

Ein Antiplasma ist definiert als Plasma, welches durch Immunisieren eines Säugetieres oder Menschen mit Antigenen erhalten werden kann. Dieses ist durch Abtrennung der korpuskulären Be- standteile des Vollblutes erhalten. Ein Beispiel für ein Antiplasma ist ein Inhibitorplasma. Ein Antiserum wird ebenso aus einem immunisierten Säugetier oder Mensch erhalten, jedoch wird dabei die flüssige Phase des Blutes nach erfolgter Gerinnung gewonnen.

Die Aufarbeitung der Antikörperfraktion kann in einer einfachen Reinigung bestehen, in manchen Fällen wird es aber angezeigt sein, eine umfassende Reinigung der Antikörper vorzunehmen, insbesondere, wenn das Versuchstier, bei welchem die Blutgerinnungsstörung induziert werden soll, empfindlich auf Verunreinigungen bzw. unspezifische Antikörper reagiert. Weiters kann die Aufarbeitung Schritte umfassen, bei welchen Substanzen, die die Wirkung der Antikörper stören könnte (beispielsweise Blutfaktoren des immunisierten Tieres), in der Präparation inaktiviert oder entfernt werden, die wesentlich stabileren Antikörper jedoch unbeschadet bleiben. Dieser Schritt kann beispielsweise in einer Hitzebehandlung bestehen, welche vorzugsweise bei einer Temperatur von 40 bis 80°C und während einer Zeitdauer von 1 Minute bis mehrere Stunden durchgeführt wird.

Zur selektiven Entfernung von interferierenden Substanzen aus der Antikörperfraktion eignen sich aber auch bestimmte Affinitätsmaterialien.

Um ein genau definiertes Krankheitsbild bei der induzierten Blutgerinnungsstörung in den Versuchstieren erzielen zu können, kann es notwendig sein, die hergestellte Inhibitorplasmapräparation enthaltend die anti-Plasma-Antikörper spezifisch für einen bestimmten Blutfaktor zu machen und/oder eine Kreuzreaktivität mit bestimmten anderen Blutfaktoren, welche im Material zur Immunisierung (Immunisierungsantigen) enthalten sein könnten, auszuschließen. Zu diesem Zweck kann beim Herstellungsverfahren ein Absorptionsschritt vorgesehen werden, bei welchem ein oder mehrere Antikörper bzw. Antikörpergruppen durch Inkontaktbringen mit bestimmten Proteinen absorbiert wird (werden). Die Absorption bestimmter Antikörper kann auch in vivo, also im Testtier erfolgen, und zwar durch Verabreichung bestimmter Antigene an das Testtier.

Das Fertigstellen zu einer zur Infusion in einem Säugetier geeigneten Präparation, die pharmazeutisch akzeptabel ist, erfolgt ebenfalls auf bekannte Weise und ist in jedem Fall abhängig von der Art des Säugetiers, welches zur Infusion vorgesehen ist.

Bevorzugte Säugetiere, bei welchen die Immunisierung durchgeführt wird, umfassen Schafe, Ziegen, Rinder, Schweine, Kaninchen, Meerschweinchen, Pferde, Ratten und Mäuse. Bevorzugte Säugetiere, bei welchen die Blutgerinnungsstörung induziert wird, sind ebenfalls die oben genannten, und allgemein insbesondere Leporidae und Nagetiere, wie Kaninchen, Ratten und Mäuse.

Gemäß einem weiteren Aspekt betrifft die vorliegende Erfindung die Verwendung von anti-Plasma-Antikörpern zur Herstellung einer Infusionspräparation zur Behandlung eines Säugetieres als Modell für einen Mangel an Blutfaktoren durch funktionelles Inhibieren und/oder Eliminieren von mehreren Blutfaktoren, so daß die Gerinnungszeit in vitro oder ex vivo bzw. die Blutungscharakteristik im Säugetier verändert wird. Gegebenenfalls werden dabei Antikörper gegen bestimmte Blut faktoren durch Verabrechung der Blutfaktoren an das Säugetier in vivo absorbiert.

Eine bevorzugte Verwendung ist daher dadurch gekennzeichnet, daß durch Resubstituieren mit einem oder mehreren Blut faktoren die veränderte Gerinnungszeit bzw. Blutungscharakteristik nur mehr von einem Mangel an jenem Blutfaktor bzw. Blutfaktoren abhängig ist, die nicht substituiert worden sind.

Um eine gute praktische Handhabung des erfindungsgemäßen Systems zur Verfügung zu stellen, betrifft eine weiterer Aspekt der Erfindung einen Kit zur Präparation eines Säugetieres als Tiermodell, welcher Kit
a) eine erfindungsgemäße anti-Plasma-Antikörperpräpration und
b) eine Infusionsvorrichtung für ein Säugetier enthält. Gegebenenfalls können noch
c) Proteine zur Absorption bestimmter Antikörper im Kit enthalten sein.

Gemäß einem weiteren Aspekt betrifft die vorliegende Erfindung ein Verfahren zur Bestimmung der Blutungscharakteristik eines Säugetieres, gekennzeichnet durch die Schritte
- Induzieren einer Blutung in einem Säugetier
- Sammeln von Blutfraktionen aus der Blutung
- Bestimmen des Hämoglobingehaltes der gesammelten Blutfraktionen und
- Bestimmen des kumulierten Blutverlustes und/oder der Blutungskinetik.

Bislang wurde die Blutungscharakteristik mit Methoden unter sucht, die nur eine grobe Abschätzung des Blutungsverlaufes ermöglicht haben bzw. sehr fehleranfällig waren. Beispielsweise wurde im Stand der Technik die Blutungscharakteristik durch bloßes Beobachten der Blutungsdauer bestimmt oder das Ende der Blutung durch kontinuierliches Abtupfen auf ein Filterpapier bestimmt (Ende = keine Blutspuren am Filterpapier mehr ersichtlich). Bei einer anderen Methode wurde das ausgetretene Blut über die gesamte Dauer gesammelt und beispielsweise durch Wägen quantifiziert, wobei stets Verdunstungsverluste miteinkalkuliert werden mußten.

Gemäß der vorliegenden Erfindung werden jedoch die aus der induzierten Wunde austretenden Blutfraktionen über die gesamte Zeitdauer der Blutung vorzugsweise kontinuierlich gesammelt, vorzugsweise indem das Blut auf geeignete Absorptionsmaterialien aufgebracht wird, und anschließend der Hämoglobingehalt der Blutfraktionen bestimmt. Als geeignete Absorptionsmaterialien haben sich beispielsweise Filtermaterialien oder Wattepfropfen erwiesen. Wichtig ist, daß sie in der Lage sind, das Blut quantitativ aufzunehmen und die Hämoglobinbestimmung nicht beeinträchtigen. Beim Sammeln der Blutfraktionen mit Absorptionsmaterialien ist darauf zu achten, daß keine Kapillarwirkungen zur Wunde hin entstehen, wodurch ein entstehendes Blutgerinnsel verletzt werden könnte.

Durch die Bestimmung des Hämoglobins, welche bevorzugterweise spektrophotometrisch erfolgt, wird die erfindungsgemäße Methode unabhängig von Verdunstungsverlusten und kann somit ein unverfälschtes und quantitativ exaktes Bild des Blutungsverlaufes liefern.

Die induzierte Blutung ist vorzugsweise eine arteriovenöse Blutung und wird hervorgerufen durch Verletzung der Extremitäten des Testtieres, wie z.B. Schnitt der Schwanzspitze oder durch Krallenschnitt (Nagelhautblutung).

Die Bestimmung der Blutungscharakteristik für das mit den erfindungsgemäßen Präparationen induzierte Tiermodell ist selbstverständlich nicht auf die Hämoglobin-Methode beschränkt, sondern kann mit jeder im Stand der Technik beschriebenen Bestimmungsmethode durchgeführt werden.

Ein weiterer Aspekt der vorliegenden Erfindung betrifft ein Verfahren zur Evaluierung von Substanzen zur Behandlung von Gerinnungsstörungen, welches die folgenden Schritte umfaßt:
- Behandeln eines Säugetieres mit einer erfindungsgemäßen anti-
   Plasma-Antikörperpräparation, so daß eine Gerinnungsstörung im Säugetier induziert wird,
- Verabreichen der zu evaluierenden Substanz an das Säugetier, und
- Bestimmung eines oder mehrerer Gerinnungsparameter und/oder
   der Änderung der Blutungscharakteristik des Säugetieres durch
   die zu evaluierende Substanz, wobei diese Bestimmung nicht oder nur teilweise am Tier selbst sondern durch separate Untersuchungen erfolgt.

Ein Gerinnungsparameter ist definiert als ein Parameter, der bestimmt wird als Aussage über den Status der Gerinnung. Die Bestimmung eines Gerinnungsparameters erfolgt nach bekannten Methoden. Beispielsweise sind eine Reihe von in vitro-Untersuchungen zur Testung von Blutgerinnungsfaktoren oder Fibrinolysefaktoren oder deren Wirkung in Blut- oder Plasmaproben bekannt.

Beim erfindungsgemäßen Verfahren wird die Blutungscharakteristik eines Säugetieres mit induzierter Blutgerinnungsstörung verglichen mit der Blutungscharakteristik eines Säugetieres mit induzierter Blutgerinnungsstörung, welches die zu testende Verbindung erhalten hat, verglichen. Es wird untersucht, inwieweit die zu testende Substanz der induzierten Blutgerinnungsstörung entgegenwirken kann. Die Bestimmung der Änderung der Blutungscharakteristik wird in der einfachsten Weise durch Vergleich der Blutungszeit zwischen den Tieren, welche die zu evaluierende Substanz erhalten haben und den Tieren, bei welchen nur die Gerinnungsstörung induziert worden ist, realisiert. Kompliziertere Bestimmungen umfassen den Vergleich des Blutungsverhaltens, den Vergleich der aus der Wunde ausgetretenen Blutvolumina, die Blutungskinetik etc.

Die zu testende Substanz kann zur Prophylaxe oder Therapie getestet werden und wird in der Regel vor oder nach der Induktion der Gerinnungsstörung verabreicht (vorzugsweise entweder gleichzeitig mit der Herbeiführung der Blutung oder sobald die infundierte Substanz im Blutkreislauf verteilt ist.

Nach Herbeiführung der Gerinnungsstörung wird vorzugsweise eine Kontrollblutung am Tier induziert, z.B. durch den Schnitt einer Kralle. Nachdem sich die Blutungscharakteristik erwartungsgemäß verändert zeigt, kann das Tier als geeignet für weitere Untersuchungen beurteilt werden.

Es ist wichtig hervorzuheben, daß die Bestimmung der Änderung der Blutungscharakteristik nicht oder nur teilweise am Säugetier selbst durchgeführt wird, sondern zumindest, was die Bestimmung von Blutfraktionen oder die Bestimmung der Gerinnungsparameter anbelangt, in separaten Laboruntersuchungen vorgenommen wird. Diese Laboruntersuchungen können Hämoglobinbestimmungen, Volumen- bzw Gewichtsbestimmungen oder ähnliche Untersuchungen sein. Weitere Untersuchungen betreffen die Beurteilung des Gerinnungssystems in Blutproben durch bekannte Methoden, z.B. aPTT, Prothrombinzeit, Bestimmung von Gerinnungsfaktoren etc.

Gemäß einem weiteren Aspekt betrifft die vorliegende Erfindung auch ein Säugetier mit einer durch eine erfindungsgemäße anti-Plasma-Antikörperpräparation induzierten Blutgerinnungsstörung, wobei die induzierte Blutgerinnungsstörung vorzugsweise ein induziertes von Willebrand-Jürgens-Syndrom oder eine andere der Hämophilie verwandte Blutungsneigung ist.

Die erfindungsgemäßen anti-Plasma-Antikörperpräparationen können nicht nur zur Induktion einer Blutgerinnungsstörung in einem Säugetier verwendet werden, sondern auch direkt zur Herstellung einer pharmazeutischen Präparation zur Vorbeugung und Behandlung von Krankheitsbildern, die mit dem Auftreten von unerwünscht hohen Aktivitäten von Blutfaktoren, einhergehen, z.B. eine anti-Human-von Willebrand-Faktor-Präparation zur Vorbeugung und Behandlung des hämolytischen oder urämischen Syndroms, des Adult Respiratory Distress-Syndroms (ARDS) oder der Arteriosklerose. Diese Zustände sind exemplarisch für jene mit Thrombozytenaggregation aufgrund abnormer von Willebrand-Faktor-Aktivitäten.

Die Erfindung wird anhand der nachfolgenden Beispiele und der Zeichnungsfigur, auf die sie jedoch nicht beschränkt sein soll, noch weiter erläutert.

Es zeigen: Fig. 1 den Mittelwert der Blutungscharakteristik von normalen Kaninchen (Δ, n = 92) im Vergleich zu Kaninchen, die mit von Willebrand-Faktor-Inhibitorplasma behandelt wurden (o, n = 11), wobei die strichlierten Kurven den Blutverlust pro Zeiteinheit und die durchgezogenen Kurven den kumulierten Blutverlust angeben und der Beobachtungsintervall zur Bestimmung der Blutungsintensität zwischen 10 und 20 min lag; Fig. 2 den Mittelwert der Blutungscharakteristik von normalen Mäusen (Δ, n = 10) im Vergleich zu Mäusen, die mit von Willebrand-Faktor-Inhibitorplasma vorbehandelt wurden (o, n = 10), wobei die strichlierten Kurven den Blutverlust pro Zeiteinheit und die durchgezogenen Kurven den kumulierten Blutverlust angeben und die Blutungsintensität im Beobachtungsintervall zwischen 6 und 16 min bestimmt worden ist; Fig. 3 das Studienprotokoll zur Testung der Wirkung von FEIBA in von Willebrand-Faktor-Inhibitorkaninchen; Fig. 4 die Blutungsintensität von von Willebrand-Faktor-Inhibitorkaninchen, die mit steigenden Dosen FEIBA behandelt wurden, wobei die Kontrollgruppe Puffer als Vergleich erhalten hat (strichlierte Linie: Mittelwert von 33 von Willebrand-Faktor-Inhibitorkaninchen ± Standardabweichung; punktierte Linie Mittelwert ± Standardabweichung von 92 normalen Kaninchen); Fig. 5 das Studienprotokoll zur Testung von IMMUNATE® oder rekombinantem von Willebrand-Faktor in von Willebrand-Faktor-Inhibitormäusen; Fig. 6 die Blutungsintensität von normalen Mäusen, Mäusen die mit von Willebrand-Faktor-Inhibitor vorbehandelt wurden und die Dosis/Wirkungsbeziehung der Blutungsintensität von von Willebrand-Faktor-Inhibitormäusen nach Zugabe von steigenden Dosen Faktor VIII/von Willebrand-Faktor-Komplex (IMMUNATE®); und Fig. 7 die Blutungsintensität von von Willebrand-Faktor-Inhibitormäusen nach Gabe von rekombinantem von Willebrand-Faktor (200 RCoF E/kg) im Vergleich zu normalen Mäusen und von von Willebrand-Faktor-Inhibitormäusen.

### Beispiele

### Beispiel 1: Gewinnung eines anti-vWF Inhibitorplasmas

Eine Ziege (50-60 kg Körpergewicht, 1,5 Jahre alt) wird mit einem von Willebrand- Faktor/Faktor VIII-Komplex immunisiert. Der von Willebrand- Faktor/Faktor VIII-Komplex wird wie folgt gewonnen:

1 g Kryopräzipitat wird in 35 ml einer Tris/Lysin-gepufferten NaCl-Lösung (85 mM) gelöst. Proteine des Prothrombinkomplexes werden durch Adsorption an AlOH₃ und BaSO₄ entfernt. Dazu wird die von Willebrand-Faktor/Faktor VIII enthaltende Lösung mit 0,1 Gew.-% AlOH₃ 1 Stunde bei 22°C inkubiert. Anschließend wird der AlOH₃-Proteinkomplex durch Zentrifugation abgetrennt. Der gewonnene Überstand wird weiters mit 0,1 Gew.-% BaSO₄ unter Rühren inkubiert und der BaSO₄-Proteinkomplex neuerlich durch Zentrifugation abgetrennt. Der Überstand wird anschließend auf pH 6,8 eingestellt und durch Chromatographie über Q-Sepharose® FF (Pharmacia) gereinigt. Der von Willebrand-Faktor-Komplex wird an das in einer Säule gepackte Gel adsorbiert; nicht-bindende Proteine werden durch Waschen mit 4 Säulenvolumina des Startpuffers entfernt. Die Elution der Proteinfraktion wird mit einem Puffer (50 mM Tris/HCl, 500 mM NaCl, pH 6,8) durchgeführt. Das gewonnene Eluat wird mit 50 mM Tris/HCl-Puffer, pH 6,8, auf eine Konzentration von 175 mM NaCl verdünnt und anschließend durch Gelfiltration auf Sepharose® CL6B (Pharmacia) weiter gereinigt. Die Gelfiltration wird mit einem linearen Fluß von 5,7 cm/h in einer Säule mit 26 mm Durchmesser und einer Betthöhe von 82 cm durchgeführt. Die Proteinfraktion des Ausschlußvolumens wird bei 280 nm UV-spektroskopisch detektiert. Der Protein-Peak des Ausschlußvolumens wird durch Ultrafiltration, mit einem Ultrafilter mit einer Retentionsmasse von 30.000 D konzentriert. Ein so hergestellter von Willebrand-Faktor/Faktor VIII-Komplex hat eine spezifische Aktivität von 173 Ristocetin-Cofaktoreinheiten (E RCoF) vWF/mg Protein (bestimmt mittels Proteinbestimmung nach Bradford, Anal.Biochem. 72:248-254, 1976). Die Präparation ist frei an Gerinnungsfaktoren II, VII, IX, X, XI und XII.

Für die Immunisierung der Ziege werden 2 mg von Willebrand-Faktor/Faktor VIII-Komplex mit 0,5 ml komplettem Freund'schen Adjuvans emulgiert und subkutan verabreicht. Vier Wochen nach der Grundimmunisierung wird ein Booster mit derselben Antigenmenge, allerdings emulgiert in inkomplettem Freund'schen Adjuvans, durchgeführt. Blutproben der Ziege werden verwendet, um den Immunisierungserfolg zu testen. Weitere vier Wochen nach dem Booster wird ein zweiter Booster mit derselben Menge Antigen durchgeführt, die Immunisierung erfolgt jedoch intravenös. Anschließend wird das Inhibitorplasma durch Plasmapherese gewonnen. Ein so gewonnenes Plasma stellt ein hochtitriges von Willebrand-Faktor-Inhibitorplasma dar, wie in den nachfolgenden Beispielen erläutert wird.

### Beispiel 2: Immunabsorption des Inhibitorplasmas

Um das nach Beispiel 1 hergestellte Inhibitorplasma spezifisch für von Willebrand-Faktor zu machen und eine Kreuzreaktivität mit Faktor VIII, der im Immunisierungs-Antigenkomplex auch enthalten sein könnte, auszuschließen, wird das Plasma mit Faktor VIII absorbiert. Dazu wird das Plasma in gleichen Volumsteilen mit einem hochreinen Faktor VIII-Präparat (Kogenate®, Miles/Cutter, 100 E FVIII/ml) 2 Stunden bei 37°C inkubiert. Das sich bildende Präzipitat wird durch Zentrifugation bei 10.000 g 10 Minuten abgetrennt. Die überstehende, Antikörper enthaltende Lösung, ist frei von Kreuzreaktivität mit Faktor VIII.

### Beispiel 3: Alternativmethode zur Gewinnung eines anti-vWF-Inhibitorplasmas

### Analog zu Beispiel 1 wird eine Ziege mit einem von Willebrand-Faktor immunisiert.

Die Herstellung des Immunisierungsantigens erfolgt wie in Beispiel 1 beschrieben, jedoch mit folgender Modifikation. Nach Gelfiltration und Ultrakonzentration des von Willebrand-Faktor-Komplexes wird dieser in einem Puffer (50 mM Glycin, 175 mM NaCl, 250 mM CaCl₂, pH 6,8) aufgenommen und durch neuerliche Gelfiltration über Sepharose®CL6B (Pharmacia) mit einem linearen Fluß von 5,7 cm/h bei 4°C auf einer Trennsäule mit 26 mm Durchmesser und 82 cm Betthöhe weitergereinigt. Dabei wird von Willebrand Faktor und Faktor VIII quantitativ dissoziiert und durch das chromatographische Trennmedium getrennt. Der Protein-Peak des Ausschlußvolumens, der durch Messung der UV-Adsorption bei 280 nm detektiert wird, wird gesammelt und durch Ultrafiltration über eine Membran mit einer Retentionsmasse von 30.000 D konzentriert. Die so gewonnene von Willebrand-Faktor-Präparation weist eine spezifische Aktivität von mindestens 200 Ristocetin-Cofaktor (RCoF) E/mg Protein auf und ist frei von Gerinnungsfaktoren II, VII, VIII, IX, X, XI und XII. Die Immunisierung und Gewinnung des entsprechenden Inhibitorplasmas erfolgt gemäß Beispiel 1.

### Beispiel 4: In vitro-Charakterisierung des von Willebrand-Faktor- Inhibitorplasmas

Das gemäß Beispiel 1 hergestellte von Willebrand-Faktor-plasma wird auf sein Kreuzreaktivität mit Maus-von Willebrand- Faktor untersucht, indem Ziegen anti-von Willebrand-torplasma mit Maus-Normalplasma, welches aus einem Pool von minde stens 10 gesunden Mäusen hergestellt wird, in einer Konzentration von 10 Vol.-% bei 37°C inkubiert wird. Vor Inkubation wird das Ziegenplasma 1 Stunde bei 56°C erhitzt, um endogen enthaltenen Ziegen-Faktor VIII, der mit dem nachfolgenden Test interferieren kann, zu inaktivieren. Proben aus der Testmischung werden unmittelbar nach Mischung der Plasmen sowie nach 1 und 3 Stunden entnommen.

Da im Mausplasma ein funktioneller von Willebrand-Faktor-Test nicht zur Verfügung steht, wird indirekt die Instabilität des Faktor VIII bei langdauernder Inkubation, die durch Fehlen des von Willebrand-Faktors zustande kommt, untersucht. Die Resultate sind Tabelle 1 zu entnehmen.

**Tabelle 1**

| **Mischung** | | **FVIII-Gehalt (%)** | |
|---|---|---|---|
| Mausplasma + | Inkubationszeit (h) | 2-Stufen Gerinnung | chromogen |
| Ziegen-vWF-Inhibitorplasma | 0 | 85 | 79 |
| | 1 | 20 | 10 |
| | 3 | 17 | 2 |
| Ziegen-Normalplasma | 0 | 76 | 105 |
| | 1 | 61 | 68 |
| | 3 | 68 | 70 |

Normales Mausplasma (= 100 %) enthält 0,3 IE FVIII/ml gemessen im 2-Stufen-Test und 0,6 IE FVIII/ml gemessen mit einem chromogenen Faktor VIII-Test (Immunochrom FVIII:C, Immuno). Durch Inkubation mit dem Inhibitorplasma nimmt die Faktor VIII-Aktivität nach 3 Stunden auf ca. 2-17 %, je nach Testmethode, ab. Als Kontrolle wird Mausplasma mit 10 Vol.-% Ziegennormalplasma inkubiert. Dabei zeigt sich keine signifikante Abnahme der FVIII-Konzentration über die Zeit.

### Beispiel 5: In vivo-Evaluierung des von Willebrand-Faktortorplasmas in Kaninchen

Weiße Neuseelandkaninchen (ca. 2 kg Körpergewicht, 6 Monate alt) werden mit Ketaminhydrochlorid (65 mg/kg) und Xylazin (10 mg/kg) narkotisiert. Die Narkose wird durch Gabe von Pentobarbital über die Versuchsdauer aufrecht erhalten. Die Tiere werden auf dem Rücken liegend plaziert, so daß die Hinterpfoten auf die Seite zum Liegen kommen. Anschließend wird die rechte Femoralvene herauspräpariert und ein venöser Zugang etabliert, der zur kontinuierlichen Infusion von Testlösungen mittels eines Perfusors VI (Braun & Melsungen AG) dient. Anschließend wird die Nagelhautblutungszeit nach einer modifizierten Methode nach Giles et al., Blood 60:727-730, 1982, bestimmt. Dazu wird das Fell um die Kralle rasiert, um zu verhindern, daß bei der späteren Blutung austretendes Blut vom Fell absorbiert wird. Die Nagelhaut wird mittels einer Krallenzange verletzt. Unmittelbar danach werden Filter (Pipetman P5000-Schutzfilter, Gilson) unter der Wunde so etabliert, daß das Blut direkt auf den Filter tropfen kann, ohne von diesem durch Kapillarwirkung aufgesogen zu werden, um zu verhindern, daß ein sich formierendes Blutgerinnsel verletzt wird. Die Filtereinheiten werden alle 2 Minuten gewechselt und das austretende Blut in Fraktionen gesammelt. Die Blutsammlung wird 30 Minuten fortgesetzt. Danach wird, sofern die Blutung nicht zum Stillstand gekommen ist, die Wunde verödet. Verschiedene Krallen können bei ein und demselben Tier verwendet werden. Als interne Kontrolle wird jeweils die Blutung vor Gabe des Inhibitorplasmas getestet.

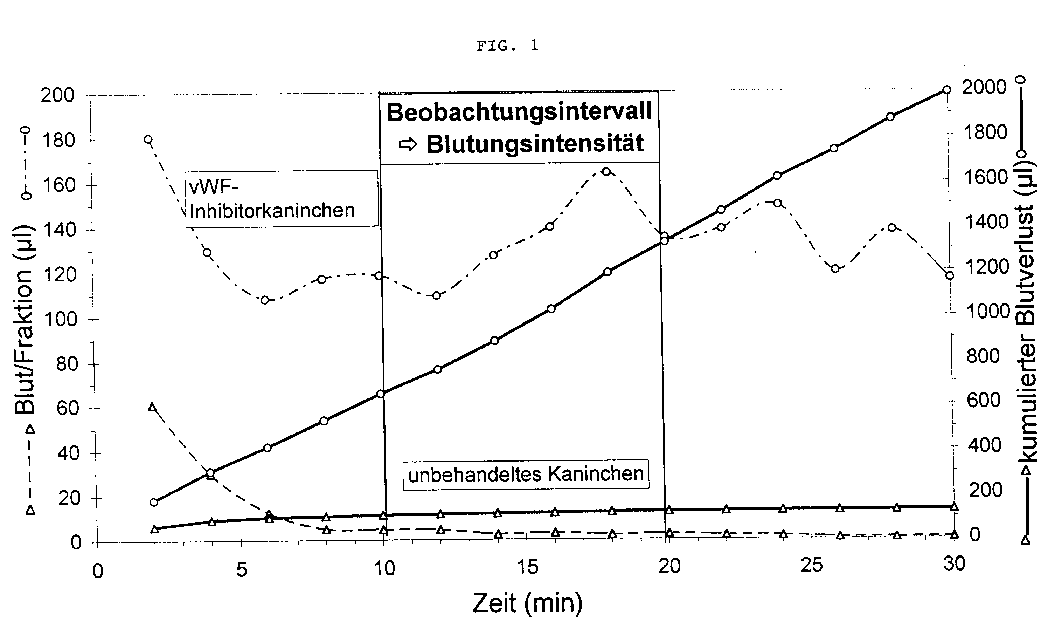

Die Qualifizierung der Blutungscharakteristik erfolgt durch Extraktion des in Fraktionen auf den Filtern gesammelten Blutes mit jeweils 5 ml 0,04 % Ammoniumhydroxid-Lösung über 5 Stunden. Dabei lysieren die Erythrozyten, die mit dem Blut im Filter gesammelt wurden. Durch eine 10-minütige Ultraschallbehandlung (Sonorex RK 100, Bandelin electronic, Berlin) wird das Hämoglobin extrahiert und quantitativ photometrisch bei 416 nm gegen eine Eichkurve bestimmt. Eine Eichkurve kann ermittelt werden, indem Kaninchenblutvolumina zwischen 10 µl und 1 ml auf die Filter pipettiert werden, diese wie oben beschrieben extrahiert werden und das Hämoglobin photometrisch bei 416 nm bestimmt wird. Entsprechend lassen sich lineare Eichkurven erstellen, die eine direkte Umrechnung der Hämoglobinkonzentration auf die Blutmenge pro Filter ermöglichen. Die Blutungscharakteristik des Nagelschnittes wird ermittelt, indem graphisch die Blutmenge pro 2-Minuten-Fraktion gegen die Zeit aufgetragen wird (Fig. 1). Zur Beurteilung der Blutungscharakteristik wird ferner der kumulierte Blutverlust ermittelt, indem die einzelnen Blutfraktionen additiv gegen die Zeit in die Graphik eingetragen werden (siehe Fig.1). Als für die Blutung relevantes Kriterium wird die Steigung der kumulierten Blutung zwischen 10 und 20 Minuten herangezogen. Dieser Wert ist unabhängig von der initialen Blutmenge, die durch schlecht standardisierbare Krallenschnittechniken variieren kann. Die Steigung dieser Blutungscharakteristik im 10-20 Minuten Beobachtungsintervall dient als Maß für die Intensität der Blutung. Eine Steigung gleich O bedeutet, daß die Blutung zum Stillstand gekommen ist; eine Steigung größer als Null mit einem Korrelationskoeffizienten von > 0,8 bedeutet, daß eine konstante Blutung vorliegt. Normale Kaninchen, die unter diesen Bedingungen getestet wurden zeigen im Beobachtungszeitraum keine Blutung mehr, d.h. die Blutung ist bereits zuvor zum Stillstand gekommen.

Analog wird Kaninchen das von Willebrand-Faktor-Inhibitorplasma, das nach Beispiel 1 oder Beispiel 3 hergestellt wurde, in einer Konzentration von 0,5 ml/kg Körpergewicht infundiert. Nach Infusion des Inhibitorplasmas zeigen die Tiere eine Blutungscharakteristik von 30-110 µl Blut/min im Beobachtungszeitraum mit einem Korrelationskoeffizienten der Blutungscharakteristikkurve von > 0,9.

### Beispiel 6: In vivo-Evaluierung des Inhibitorplasmas in der Maus

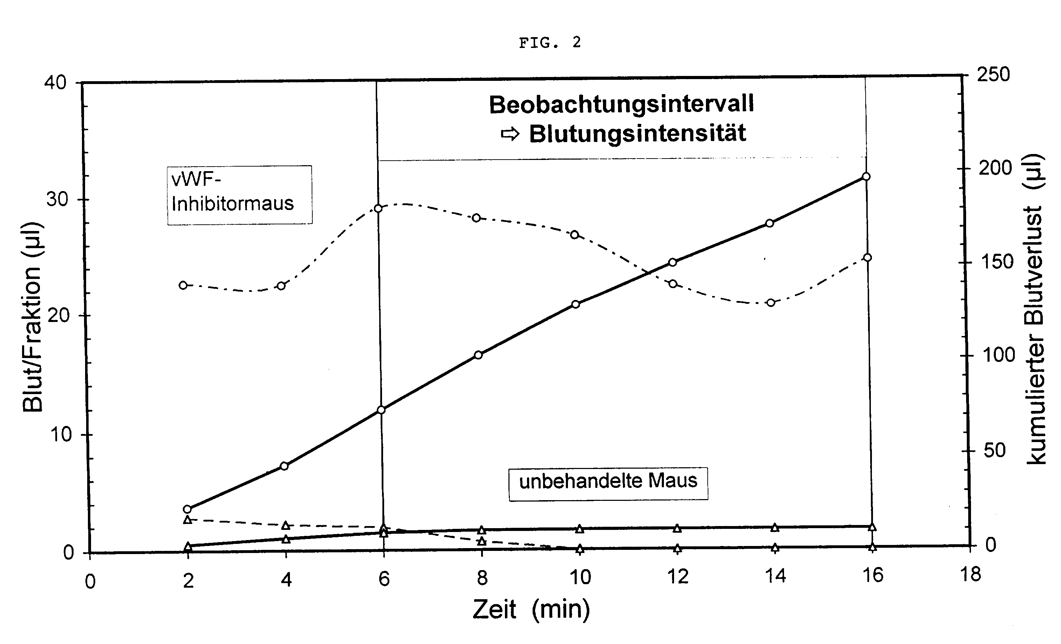

Weibliche NMRI-Mäuse (20-30 g) werden verwendet und durch Gabe von 63 mg/kg Pentobarbital narkotisiert. Anschließend wird die Blutungscharakteristik mit einer modifizierten Methode nach Novak et al., Br.J.Haematol. 69:371-378, 1988, durch definierte Verletzung der Schwanzspitze bestimmt. Wie im Beispiel 5 beschrieben wird Blut, welches aus der arteriovenösen Verletzung austritt, auf Filtern gesammelt und analog zu Beispiel 5 quantitativ bestimmt. Im Gegensatz zu den Kaninchen, wo mehrere Krallen zum Schnitt zur Verfügung stehen, kann hier keine interne Kontrolle im Bezug auf das Normalblutungsverhalten durchgeführt werden. Daher wird eine Kontrollgruppe an Mäusen mit 0,9 %iger NaCl-Lösung (2 ml/kg Körpergewicht) behandelt. Die Blutungscharakteristik der Kontrollgruppe ist Fig. 2 zu entnehmen. Der Meßzeitraum beträgt hier 16 Minuten, das Beobachtungsintervall liegt zwischen 6 und 16 Minu ten. Unbehandelte Mäuse weisen im Beobachtungsintervall eine Blutungscharakteristik von < 0,01 µl Blut/min auf. Analog zu Beispiel 5 werden Mäuse mit 10 ml/kg anti-von Willebrand-Faktor-plasma behandelt und anschließend das Blutungsverhalten quantifiziert. Mit von Willebrand Faktor-Inhibitorplasma gemäß Beispiel 1 oder 3 behandelte Mäuse weisen eine Blutungscharakteristik von 4-19 µl Blut/min auf. Der Korrelationskoeffizient der die Blutungscharakteristik bestimmenden Kurve liegt bei > 0,8. Nach Abschluß des Versuches wird den Mäusen Blut durch Herzpunktion entnommen, mit Citrat versetzt und Plasma gewonnen. Aus diesem Plasma wird die Struktur des von Willebrand-Faktors nach der Methode von Ruggeri et al., Blood 57:1140-1143, 1981, bestimmt. Dabei werden die von Willebrand-Faktor-Multimere durch eine immunenzymatische Färbung nach Aihara et al., Thromb.Hämostas. 55: 263-267, 1986, bestimmt. Als primärer Antikörper wird ein Kaninchen anti-human von Willebrand-Faktor-Antiserum (Dakopatts) in einer Verdünnung von 1:5000 verwendet. Als sekundärer Antikörper dient ein alkalische Phosphatase-konjugierter affinitätsgereinigter Ziegen anti- Kaninchen IgG (H+L)-Antikörper (Axell, Accurate Chemical and Scientific Corp., NY) in einer Verdünnung von 1:1000. Die Färbung der Proteinbanden erfolgt mittels des Nitroblautetrazoliumchlorid/ Bromchlorindolylphosphat-Substrat-Systems.

Maus-Normalplasma weist unter diesen Testbedingungen eine charakteristische von Willebrand-Faktor-Multimerenstruktur auf. Nach Gabe des von Willebrand-Faktor-Inhibitors ist in einer Reihe von 5 untersuchten Mäusen bei 3 kein von Willebrand Faktor mehr nachweisbar. In 2 aus 5 Tieren ist der von Willebrand-Faktor-Gehalt deutlich reduziert, von Willebrand-Faktor-Multimere sind nur mehr schwach sichtbar.

### Beispiel 7: Testung der Wirksamkeit von FEIBA an Kaninchen, die mit von Willebrand-Faktor-Inhibitor vorbehandelt wurden.

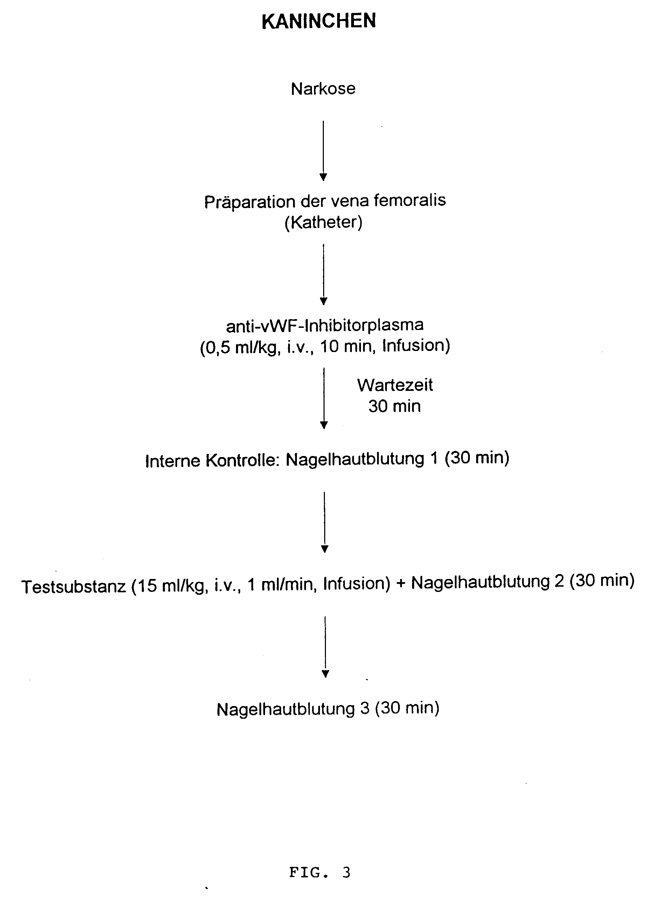

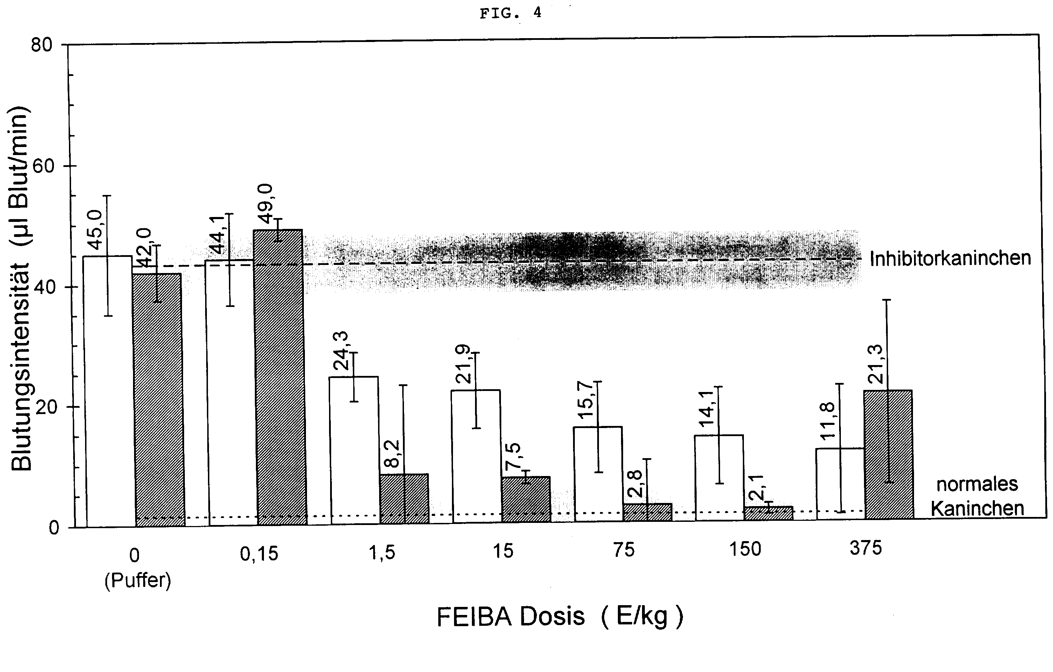

Das Studienprotokoll ist Fig. 3 zu entnehmen. Kaninchen erhielten 0,5 ml/kg von Willebrand-Faktor-Inhibitorplasma, welches gemäß Beispiel 1 hergestellt wurde. FEIBA S-TIM4, Immuno, (1000 E) wurde mit 20 ml A. dest. rekonstituiert. Nach vollständiger Lösung der Substanz wurde diese mit Citratpuffer (4 g Na₃.Citrat, 1,8 g NaCl pH 7,0) verdünnt und den Kaninchen 30 ml der jeweiligen Verdünnungen mit einer Infusionsgeschwindigkeit von 1 ml/min infundiert, so daß sich Dosen von 0,15, 1,5, 15, 75, 150 und 375 E/kg ergaben. Die Blutungscharakteristik wurde parallel mit der Substanzinfusion und 30 min nach Beginn der FEIBA-Infusionen wie in Beispiel 5 beschrieben bestimmt. Fig. 4 zeigt den Median der Blutungsintensität im Beobachtungsintervall von 6 Kaninchen/Gruppe. Die Blutungsintensität der Nagelhautblutung, die gleichzeitig mit der Substanzinfusion durchgeführt wurde, zeigt eine dosisabhängige Reduktion mit einem Maximum bei 375 E/kg. Nach Abschluß der FEIBA-Infusion zeigt die Blutungscharakteristik ein Intensitätsoptimum zwischen 75 und 150 E/kg. Von diesem Dosisbereich ist das Blutungsverhalten unbehandelter Kaninchen nicht zu unterscheiden. Bei einer Dosis von 375 E/kg ist der hämorrhagische Effekt wieder verstärkt. Dieses Ergebnis zeigt, daß mit FEIBA, gemäß der Indikation, ein Effekt, der durch einen Gerinnungsinhibitor hervorgerufen wird, neutralisiert werden kann. Die in der humanen Anwendung als optimale Wirkungsdosis für Blutungen empfohlene Dosis von 50-100 E FEIBA/kg wird durch das Modell bestätigt. Damit ist die Relevanz des Tiermodells für Kaninchen mit von Willebrand-Faktor-Inhibitor gezeigt.

### Beispiel 8: Effekt eines Faktor VIII/von Willebrand-Faktor-Komplexes in Mäusen, die mit einem von Willebrand Faktor-Inhibitorplasma vorbehandelt wurden

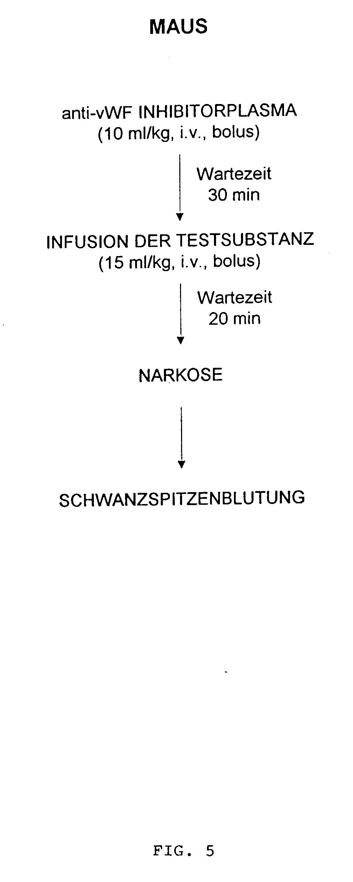

Der Studienplan ist der Fig. 5 zu entnehmen.

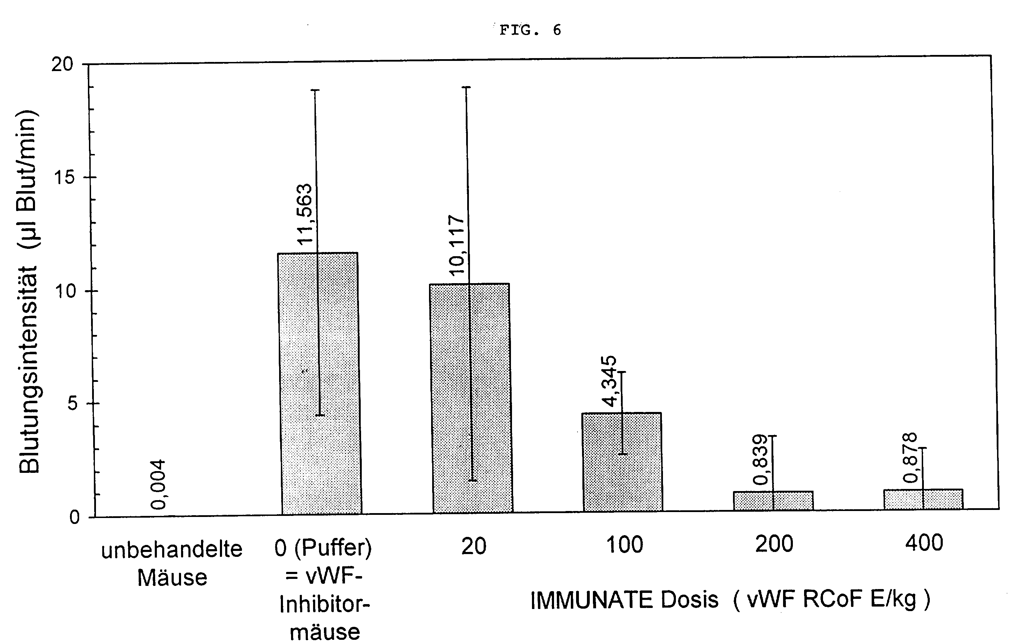

Mäuse wurden mit anti-von Willebrand Faktor-Inhibitor, der in Beispiel 3 beschrieben ist, mit einer Dosis von 10 ml/kg Körpergewicht vorbehandelt. Fig. 6 zeigt, daß die Blutungsintensität einer Gruppe von 10 Mäusen, die mit anti-von Willebrand-Faktor-Inhibitorplasma vorbehandelt wurde, im Mittel 11,6 µl Blut/min beträgt. Unbehandelte Mäuse zeigen im Vergleich dazu eine Blutungsintensität von 0,004 µl/min im Beobachtungsintervall. Immunate S-TIM4, Immuno, (250 E/Fl.) wurde gemäß der Herstellerangabe mit A.dest. rekonstituiert, so daß die Lösung 10,9 Ristocetin-Cofaktoreinheiten (E RCoF) vWF/ml und 50 IE FVIII/ml enthielt. In einem konstanten Dosisvolumen von 20 ml/kg Körper gewicht wurden den Mäusen Dosen von 20, 100, 200 und 400 Ristocetin-Cofaktoreinheiten (E RCoF) vWF/kg Körpergewicht infundiert. Fig. 6 zeigt die Blutungsintensität des Mittelwertes von jeweils 10 Tieren/Gruppe. Die Blutungsintensität konnte dosisabhängig reduziert werden, wobei mit 200 Ristocetin-ten (E RCoF) vWF/kg Körpergewicht das Blutungsverhalten der hämorrhagischen Mäuse nahezu normalisiert werden konnte. Die Multimerenanalyse, die wie in Beispiel 5 beschrieben, an Plasmaproben von von Willebrand-Faktor-inhibierten Mäusen nach Immunate-Gabe durchgeführt wurde, zeigt, daß der von Willebrand Faktor, der die abnorme Blutungsneigung kompensieren konnte, der infundierte humane von Willebrand-Faktor/Faktor VIII-Komplex war, und nicht der endogene Maus-von Willebrand-Faktor, der aus den Immunkomplexen hätte freigesetzt werden können. Damit ist die Effizienz des Modells für die Evaluierung von Substanzen, die bei von Willebrand-Faktor-Inhibitoren eingesetzt werden, gezeigt.

### Beispiel 9: Effekt eines rekombinanten von Willebrand-Faktor-Präparates in Mäusen, die mit einem von Willebrand-Faktor-Inhibitorplasma vorbehandelt wurden

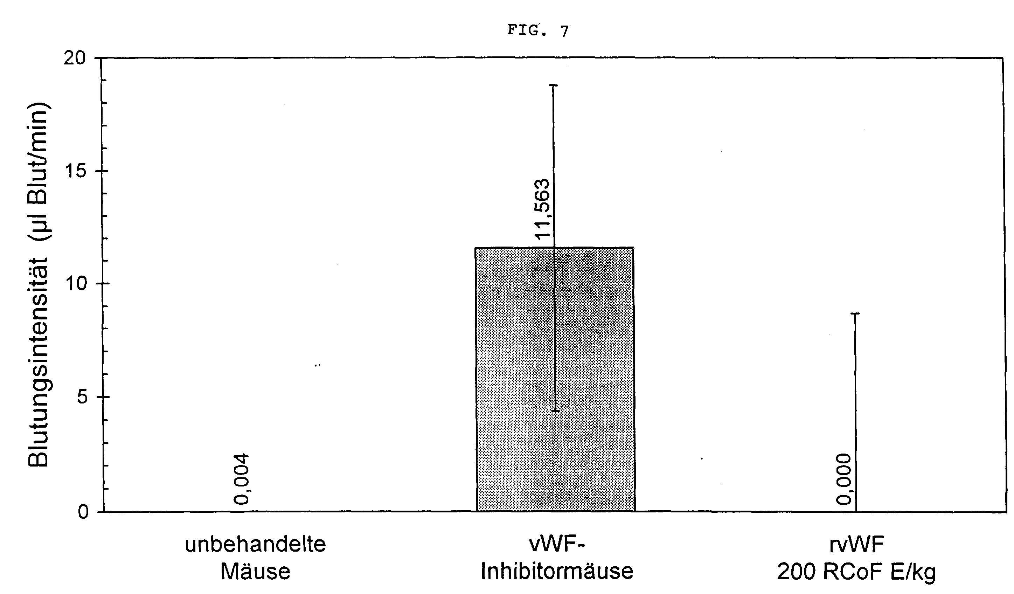

Analog zum Beispiel 8 wurden Mäuse mit einem rekombinanten humanen von Willebrand-Faktor, der aus CHO-Zellen gewonnen worden war, mit einer Dosis von 200 Ristocetin-Cofaktoreinheiten (E RCoF) rvWF/kg Körpergewicht behandelt. Fig. 7 zeigt die Blutungscharakteristik von unbehandelten Mäusen im Vergleich zu von Willebrand-Faktor- Inhibitor-vorbehandelten Tieren, die mit rekombinantem von Willebrand-Faktor behandelt wurden. Durch Gabe von 200 Ristocetin-Cofaktoreinheiten (E RCoF) rvWF/kg Körpergewicht konnte das abnorme Blutungsverhalten der von Willebrand-Faktor-Inhibitormäuse auf das von gesunden Mäusen korrigiert werden. Durch Verwendung einer reinen rekombinanten Präparation eines von Willebrand-Faktor-Proteins, die mit anderen Plasmaproteinen aufgrund der Herkunft nicht verunreinigt sein kann, wird bestätigt, daß es sich bei dem Modell um ein von Willebrand-Faktor-spezifisches Tiermodell handelt.

## Patentansprüche

1. Anti-Plasma-Antikörper-Präparation zur Behandlung an einem Säugetier, welche Präparation mehrere Blutfaktoren direkt oder indirekt inhibieren und/oder eliminieren kann.

2. Präparation nach Anspruch 1, dadurch gekennzeichnet, daß sie Antikörper enthält, welche gegen den von Willebrand-Faktor oder den Faktor VIII/von Willebrand-Faktor-Komplex gerichtet sind und sowohl den von Willebrand-Faktor als auch den faktor VIII funktio-nell inhibieren und/oder eliminieren.

3. Anti-Plasma-Antikörper-Präparation, welche erhältlich ist durch Immunisieren eines Säugetiers mit Plasma oder einer Plasmafraktion oder mit deren rekombinantem Äquivalent, Gewinnen des Antiplasmas oder Antiserums und anschließender Absorption von einem oder mehreren Antikörpern, so daß die Präparation nur solche funktionellen Antikörper enthält, die selektiv einen oder mehrere ausgewählte Blutfaktoren in einem Säugetier funktionell inhibieren und/oder eliminieren können.

4. Präparation nach Anspruch 3, dadurch gekennzeichnet, daß die Immunisierung mit Faktor VIII/von Willebrand-Faktor-Komplex erzielt wird und die Absorption von Faktor VIII-Antikörpern durch Zugabe von Faktor VIII erfolgt oder die Absorption von von Willebrand-Faktor-Antikörpern durch Zugabe von von Willebrand-Faktor erfolgt.

5. Verfahren zur Herstellung einer Präparation nach einem der Ansprüche 1 bis 4, gekennzeichnet durch die folgenden Schritte:
- Immunisieren eines Säugetieres mit Plasma oder einer Plasmafraktion oder mit deren rekombinantem Äquivalent,
- Gewinnen des Antiplasmas oder Antiserums,
- gegebenenfalls Aufbereiten der Antikörperfraktion aus dem Antiplasma oder Antiserum,
- gegebenenfalls Absorbieren von einem oder mehreren Antikörpern durch in Kontakt bringen der Antikörper mit bestimmten Proteinen und
- Fertigstellen zu einer zur Infusion in einem Säugetier geeigneten Präparation

6. Verwendung von Anti-Plasma-Antikörpern zur Herstellung einer Infusionspräparation zur Behandlung eines Säugetieres als Modell für einen Mangel an Blutfaktoren durch funktionelles Inhibieren und/oder Eliminieren von mehreren Blutfaktoren, so daß die Gerinnungszeit in vitro oder ex vivo bzw. die Blutungscharakteristik im Säugetier verändert wird.

7. Verwendung nach Anspruch 6, dadurch gekennzeichnet, daß durch Resubstituieren mit einem oder mehreren Blutfaktoren die veränderte Gerinnungszeit bzw. Blutungscharakteristik nur mehr von einem Mangel an jenem Blutfaktor bzw. Blutfaktoren abhängig ist, die nicht substituiert worden sind.

8. Kit zur Präparation eines Säugetieres als Tiermodell enthaltend
a) eine Präparation nach einem der Ansprüche 1 bis 4 und
b) eine Infusionsvorrichtung für ein Säugetier und gegebenenfalls
c) Proteine zur Absorption bestimmter Antikörper.

9. Verfahren zur Bestimmung der Blutungscharakteristik eines Säugetiers, gekennzeichnet durch die folgenden Schritte
- Induzieren einer Blutung in einem Säugetier,
- Sammeln von Blutfraktionen aus der Blutung,
- Bestimmen des Hämoglobingehaltes der gesammelten Blutfraktionen und
- Bestimmen des kumulierten Blutverlustes und/oder der Blutungskinetik.

10. Verfahren nach Anspruch 9, dadurch gekennzeichnet, daß das Sammeln der Blutfraktionen auf geeigneten Absorptionsmaterialien, vorzugsweise auf Filtermaterialien, erfolgt.

11. Verfahren zur Bestimmung der Blutungscharakteristik eines Säugetiers, gekennzeichnet durch die folgenden Schritte
- Herbeiführen einer Blutung in einem Säugetier, bei welchem eine Blutgerinnungsstörung mit einer Präparation nach einem der Ansprüche 1 bis 4 herbeigeführt worden ist,
- Sammeln von Blutfraktionen aus der Blutung,
- Bestimmen des kumulierten Blutverlustes und/oder der Blutungskinetik anhand der gesammelten Blutfraktionen.

12. Verfahren nach Anspruch 11, dadurch gekennzeichnet, daß das Sammeln der Blutfraktionen auf geeigneten Absorptionsmaterialien, vorzugsweise auf Filtermaterialien, erfolgt.

13. Verfahren nach Anspruch 11 oder 12, dadurch gekennzeichnet, daß der Hämoglobingehalt der kontinuierlich gesammelten Blutfraktionen bestimmt wird.

14. Verfahren zur Evaluierung von Substanzen zur Behandlung von Gerinnungsstörungen, gekennzeichnet durch die folgenden Schritte:
- Behandeln eines Säugetieres mit einer Präparation nach einem der Ansprüche 1 bis 4, so daß eine Gerinnungsstörung induziert wird,
- Verabreichen der zu evaluierenden Substanz an das Säugetier, und
- Bestimmung der Änderung eines oder mehrerer Gerinnungsparameter und/oder der Blutungscharakteristik des Säugetieres durch die zu evaluierende Substanz, wobei diese Bestimmung nicht oder nur teilweise am Tier selbst, sondern durch separate Untersuchungen erfolgt.

15. Säugetier mit einer durch eine Präparation nach einem der Ansprüche 1 bis 4 induzierten Blutgerinnungsstörung.

16. Säugetier nach Anspruch 15 mit einer induzierten Hämophilie, insbesondere mit einem induzierten von Willebrand-Jürgens-Syndrom.

17. Verwendung einer Präparation nach Anspruch 2 oder 4 zur Herstellung einer Präparation zur Vorbeugung und Behandlung von Zuständen mit Thrombozytenaggregation aufgrund abnormer von Willebrand-Faktor-Aktivitäten, z.B des hämolytischen urämischen Syndroms, des Adult Respiratory Distress Syndroms (ARDS) oder Arteriosklerose.
